# EUROPEAN PATENT APPLICATION

(11) **EP 4 328 769 A1**
(43) Date of publication of application: **28.02.2024**
(21) Application number: 22192498.8
(22) Date of filing: 26.08.2022
(51) Int. Cl.: G06F 21/32, G06F 21/33, G06F 21/45, G06F 21/62, G06F 21/64, G06Q 10/00

(54) **A METHOD OF DIGITALLY AUTHENTICATING A USER'S HEALTH STATUS**

(71) Applicant: Sita Advanced Travel Solutions Limited, Aldershot, Hampshire GU11 1PZ (GB)
(72) Inventor: Sanglier, Adrien, Montreal, Quebec H3A 1G1 (CA); Zureik, Michael, Montreal, Quebec H3A 1G1 (CA); Shedden, Rowan, North Sydney, NSW 2060 (AU)
(74) Representative: Wiseman, Robert George

(57) **Abstract**

A method of digitally authenticating a user's health status, the method comprising: sending a certificate representative of health data associated with a user to a verifying entity; the verifying entity carrying out verification on the certificate and, if the certificate is verified, providing a credential based on the verified certificate; and authenticating the user associated with the health data based on the credential.

## Description

### FIELD OF THE INVENTION

The present invention relates to a method of digitally authenticating a user's health status. In addition, the present invention relates to a method of digitally authenticating a user's health status performed at a user device, an authenticator, and a verifying entity.

### BACKGROUND OF THE INVENTION

Authentication of a user's health status can be beneficial and indeed be required in a number of scenarios. For example, when a user wishes to travel through a transportation hub such as an airport or train station, a user may be required to pass a health check in order to proceed. This may include proving a vaccination status or recent test result. A similar check may be made when attempting to gain access to a public venue such as a restaurant or a bar.

A health record such as a vaccine status or test result related to a user is typically verified using a number of methods. One method includes central verification typically comprising a physical or digital certificate which may be represented with an identifier such as a barcode being verified by a central verifying entity held by, for example, a lab or clinic. A user may present their identifier or barcode to a verifier such as an airport check-in desk and the result is verified remotely through the verifying entity. This method results in large amounts of vulnerable sensitive health data being stored centrally. In addition, such methods are not standardized, there exists a lack of interoperability of digital solutions, and the methods require an internet connection to perform verification.

Other existing techniques include using a proprietary application on a mobile device which may be based on a wallet on the device, and performing a look up into a decentralized database such as a distributed ledger.

It is therefore desirable to provide a method of authentication overcoming these drawbacks.

### SUMMARY OF THE INVENTION

The invention is defined by the independent claims to which reference should now be made. Optional features are set forth in the dependent claims.

Existing verification methods using central databases having sensitive information such as health data are particularly vulnerable to attacks such as DNS spoofing. A verifier may be unsure that the consulted website is the intended source of data, and an internet connection is required at the time of verification. Inventors of the present invention have appreciated the need for an ecosystem that can facilitate exchange of sensitive information between entities such as governments without a centralised database containing sensitive personal information.

The inventors of the present invention have also appreciated the need for a health status authentication method that can be performed offline while providing only the necessary health data to the relevant verifying parties. That is, health data may be represented in the form of certificates, and a verified certificate may be provided to an authenticator in the form of a credential, rather than the presentation of the health data itself on which the credential is ultimately based. This advantageously provides a zero knowledge proof, or in other words, questions regarding health data associated with a user may be answered without revealing the health data itself. This also advantageously provides a separation between trust and data in that the user is authenticated without having to explicitly provide health data to the verifier.

The inventors have yet further appreciated the ability to manage verification of a certificate representative of health data in real-time. In other words, a credential based on a verified certificate may be revoked if considered to be no longer compliant. Arrangements of the present invention provide solutions to manage a user's health status and enable governments to enforce quarantines through the issuing of credentials up to date with relevant rules. It also provides a solution for individuals to claim their healthy status and conveniently share that status in order to be able to access a service.

According to an aspect of the present invention, there is provided a method of digitally authenticating a user's health status, the method comprising: sending a certificate representative of health data associated with a user to a verifying entity; the verifying entity carrying out verification on the certificate and, if the certificate is verified, providing a credential based on the verified certificate; and authenticating the user associated with the health data based on the credential.

Health data may non-exhaustively include, for example, a test result such as a PCR test result or antigen test result, and/or proof of vaccination or vaccination status. Significantly, the certificate is representative of the health data and, in some embodiments, does not comprise the user's health data. In this way, only the necessary information is disclosed, and sensitive, vulnerable data need not be transferred between entities.

The certificate may comprise a data schema and definition describing the data fields required by health authorities and relevant government agencies to adequately prove an individual's health record status. The credential may comprise a data schema and credential definition describing the data fields required by relevant government agencies and interested parties to adequately prove an individual's trusted traveller status (that is, that they meet the authentication requirements to travel or enter a particular organisation).

The certificate and or the credential may comprise identity data associated with the user's identity. In this way, it can be verified that the user presenting the credential is the valid holder of the credential. That is, the authentication may comprise verifying that a user providing the credential is the user associated with the health data. The user's digital identity information may be biographic and/or biometric data associated with the user, or a reference to a travel document (passport, identification (ID) card, driver license, social security number, International Civil Aviation Organisation (ICAO) digital travel credential (DTC), etc.).

In some embodiments, the credential may be a verifiable credential. One or more additional credentials may be derived from a DTC embedded on the verifiable credential. The one or more additional credentials may be derived for use in subsequent verification steps in the travel continuum. For example, a hotel may not need to know the full identification information about a user as found on a passport, but only given name, surname, and passport number for example. This may be achieved due to the "selective disclosure" of a verifiable credential, a feature allowing only particular information contained within the credential as a whole to be disclosed.

Authentication is based on the credential provided following verification of the certificate. Therefore, similarly to the certificate, the credential may not contain the user's health data but instead only the relevant information needed for authentication of the user's health status. The certificate is not shared with the authenticator, only to the verifying authority, who provide the credential for authentication. In other words, the authenticator can request only the data required for authentication, and not the entire credential, thereby protecting privacy. In some embodiments, no data can be sent to the authenticator without approval from the user.

The certificate may also be termed a health credential, and the credential used for authentication of the user's health status may be termed a trusted traveller credential.

The verification by the verifying entity may be based on one or more rules. In more detail, complex rules or business rules can be implemented at the time the credential is issued. The rules may be implemented through machine readable governance which allows policies to be implemented quickly within a decentralized secure system. For example, the rules may non-exhaustively include determining whether or not one or more of: the user has purchased insurance; the user has given consent; the user has completed or more health questions; and health data has been verified.

Advantageously, providing such an authentication method enables an entity or authority to manage a credential or pass for an individual in real-time. That is, a credential may be initially authenticated in accordance with described embodiments. However, in response to a change in one or more criteria, the credential may be revoked. For example, if a data issuer is not trusted anymore (IT may have been compromised, or a process to issue health data such as a particular test or vaccination status is not compliant anymore), if a credential was issued under particular conditions which subsequently change (i.e. health data obtained from a particular clinical lab is no longer considered compliant), or if a particular vaccine is not considered effective anymore, the corresponding issued credential can be revoked immediately. In some embodiments, the credential is revoked by the verifying entity. It is particularly advantageous that an authenticating entity performing authentication needs not take any action in response to the changing criteria and revocation of the credential - the credential will be revoked such that it will not pass an authentication.

Similarly, a certificate representative of a user's health data may initially not be verified by the verifying entity and a credential may not be provided as the certificate and ultimately the user's health status do not meet the particular requirements for verification or satisfy the relevant rules. Criteria may then change, for example the requirements for health data may become lower or more lenient. The verifying entity may, in response to this change, issue the corresponding credential which may then be used for authentication. Again, the authenticating entity need not take action.

Updating or revoking a credential in real time may comprise updating at least one of the one or more rules in response to a change in one or more criteria.

While verification of the certificate and issuing of the credential have been described with respect to a verifying entity such as a government entity, it will be appreciated that the verification process may comprise an additional issuing agent carrying out one or more of the steps of issuing the credential to a user. That is, the verifying entity may comprise, or may work in conjunction with, an issuing entity or issuing agent. For example, the issuing agent may perform one or more of: displaying a unique identifier such as a QR code, or sending and receiving communication signals such as a Bluetooth signal, for a user to establish a secure connection with the issuing agent; receiving identity information about a user associated with the health data; matching information regarding the user, for example using standard correlation API that may be used to retrieve information about a user into a specific IT environment (this may be done by, but may not be limited to, a secure database lookup); formatting information following the data schema and credential definition as published into the distributed ledger; generating and issuing the credential to the user over the established secure connection; reporting on credentials issued to users; and revoking credentials upon request by the verifying entity or issuer. In the embodiments described herein, it will therefore be appreciated that where a verifying entity is described, or steps performed by a verifying entity are described, said verifying entity may comprise, or work in conjunction with, an issuing agent to carry out one or more steps of the method described.

In some embodiments, the credential may be provided to an authenticator or authenticating entity using one or more communication or media methods which may include: a unique identifier such as a QR code, Bluetooth low energy (BLE) or Bluetooth classic, Ultra-sonic (uses device's standard speaker and microphone), or Wifi Direct (ad-hoc network).

The credential data may be held on a user device, such as in the digital wallet of a user device. This advantageously enables peer-to-peer authentication of the credential, which provides offline authentication. The inventors of the present invention have appreciated that this is a much needed condition for broad adoption of decentralized identity. In order to provide offline authentication, the method may comprise the authenticator accessing an online network to cache decentralized identifiers or trusted issuers. No personal data should be stored in cache from the user's perspective. In this way, when a peer-to-peer offline connection is made between the user device and the authenticator, the authenticator has a record of trusted issuers without requiring further connection to an online network, and thus authentication may be performed offline.

In some embodiments, the authentication comprises one or more of: receiving the credential representative of the user's health data; checking the credential against a distributed ledger; decoding the credential to make sure the credential originated from a trusted source; and automating the process, meaning the authentication can be provided by the authenticator without requiring action from the verifying entity.

In some embodiments, the method comprises applying a cryptographic signature to the health data to prevent alteration of the health data. The authenticator may view the cryptographic signature of the verifying entity or issuer and know that the data has arrived unaltered, and as written to the credential. In this way, security of the health data is further improved.

The method may further comprise receiving the certificate representative of the health data associated with the user from a health data source. For example, a user may undergo one or more tests or vaccinations at a health data source such as a test centre, testing laboratory, or vaccination centre, the one or more tests or vaccinations providing the health data through test results or vaccination status. In response, the user may receive the certificate representative of the health data. The health data source such as laboratory or clinic may obtain the health data by, for example, collecting a sample from the user and linking the sample to the user, and subsequently issuing a health check record as proof of the health data such as being tested or vaccinated. This may be achieved by issuing a credential proving, for example, that a test sample has been taken. This may be a third and separate credential from the health credential and trusted traveller credentials.

The user's identity may be matched to a record such as a laboratory record to ensure the correct user is associated with the health data, and the subsequently issued certificate is associated with the correct user. In some embodiments, receiving the certificate may comprise establishing a secure link between the health data source and the user by a unique identifier associated with the user. For example, when a laboratory obtains a user's health data through a test or sample, the user may first present a unique identifier such as a QR code which may be scanned by the laboratory. In this way, confidence is provided that the health data is associated with the correct user and trust is established between the laboratory and the user.

Alternatively, a user at the laboratory may interact with a system API as follows in order to confidently associate the user with the health data. The user of the laboratory may post the following JSON to /api/test_id on the laboratory system:

```
 {"token": "abc123",
        "lab_order_id": "135231"
        "patient_first_name": "John"
        "patient_last_name": "Doe" }
```

Upon success, the API may return an HTTP 200 success code and a response body containing the patient ID in JSON format as follows:

```
 {
        "patient_id": "123x43re3" }
```

Once test results have been determined and recorded, the laboratory may post the following information to /api/tst_result. This allows the laboratory user to issue a test_result credential to the traveller as follows:

```
 { "patient_id": "123x43re3"
        "lab_order_id": "135231",
        "lab_description": 'COVID 19 PCR Test',
        "result": "Negative" }
```

All message may contain an authorization header with a base64 encoded API key. The API key may be a combination of an ID and password joined by a single ':'. The API key ID can be any collection of alphanumeric and special characters for the identification of the requestee. The API key secret may be at least 32 bytes in length, unique, random, and composed of alphanumeric and special characters.

The API Key before Base64 encoding may look like:
ApiKeyID:ApiKeySecret

This Authorization header may look like:
Authorization: Basic Base64EncodedApiKey

API Key secrets may be required to be stored via password hashing. The API may also include IP Address whitelisting. All messages may use HTTPS (TLS).

If an ID does not exist or a key is unrecognised or compromised, the response may be a HTTP code 401 message.

It will be understood by the skilled person that the described interaction with the API is merely exemplary.

Authentication may take place, for example, during a check-in process for an airline. By providing the digital credential, the authentication of the user's health status may be carried out in addition to typical check-in procedures, conveniently allowing users to complete the check-in process including a health status check remotely.

According to another aspect of the invention, there is provided a method of digitally authenticating a user's health status, performed at a user device, the method comprising the user device: sending a certificate representative of health data associated with a user; receiving a credential based on the certificate being verified; and providing the credential for authentication.

The user device may comprise any suitable electronic device such as a mobile device, tablet computer, laptop computer, or the like.

According to another aspect of the invention, there is provided a user device comprising means for digitally authenticating a user's health status, the user device comprising: means for sending a certificate representative of health data associated with a user; means for receiving a credential based on the certificate being verified; and means for providing the credential for authentication.

The user device may be configured to download, install, and run an application for performing one or more of the method steps according to techniques of this disclosure. Such an application may be configured to: receive and store minimal personal information about a user (enough to positively identify the individual to the issuer's system when conducting future interactions) which may be, for example, identity information such as passport data (this may be received via user input or through communication with a camera of the electronic device which may scan a user's passport); generate and display a unique identifier such as a QR code to establish a secure connection with a health data source; receive and store a credential issued by the health data source providing that a sample has been taken; receive and store the certificate representative of health data associated with the user; send the certificate representative of health data associated with the user to a verifying entity; receive, from the verifying entity, the credential based on the certificate being verified; provide the credential to an authenticator for authentication; and facilitate an identity check to confirm association of the user with the credential (and ultimately the health data). Secure connections are established between the user device and the verifier, and the user device and the authenticator. The secure connection may be established through the use of the Aries framework connection protocols and DIDComm messaging. Once the connection has been established, all message data exchanged may be cryptographically encoded in such a way that only the two connected parties can decrypt the messages.

The application may comprise, or may be in addition to and used in conjunction with, a digital wallet of an electronic device. For example, one or more of the certificate and credentials referenced herein may be stored, generated, and displayed using a digital wallet of an electronic device.

Thus, a user's experience is made efficient and convenient through use of an electronic device capable of facilitating the authentication of the user's health status through communication with a health data source, a verifying entity, and an authenticator.

In addition, such an application may also allow for consolidation of travel cards such as embarkation-disembarkation cards by enabling purchasing of such cards through the application. It is particularly advantageous that an authenticating entity such as an airline may verify clearance to board through the application. That is, a remote check-in process may be conveniently facilitated through the user device including health status checks.

The authenticator may utilize a corresponding application, which may similarly run on a suitable electronic device. The authenticator application may facilitate one or more of: presenting a unique identifier such as a QR code to invite users or holders to establish a secure connection with the authenticator; establishing a secure connection to a user using Aries framework connection protocols and DIDComm messaging; requesting and receiving presentation of proof attributes, as defined by data schema, published in the distributed ledger over the secure connection; authenticating the credential and ensuring data integrity and valid issuer origin through the use of cryptography and matching DIDs (Digital IDentifiers) in the distributed ledger; running business rules used for authentication; and providing a result of authentication and sending result to the user over a secure connection.

According to another aspect of the invention, there is provided a method of digitally authenticating a user's health status, performed at an authenticator, the method comprising the authenticator: receiving a credential representative of a verified certificate representing health data associated with a user; and authenticating the user associated with the health data based on the credential.

An authenticator or authenticating entity carrying out the authentication may comprise an entity such as an airline check-in desk at a transportation hub configured to receiving the credential from the user and authenticate the user based on the credential. The credential may be provided to the authenticator through one or more communication or media methods which may include: a unique identifier such as a QR code, Bluetooth low energy (BLE) or Bluetooth classic, Ultra-sonic (uses device's standard speaker and microphone), or Wifi Direct (ad-hoc network).

Alternatively, the authenticator or authenticating entity may be a remote entity. That is, authentication may be provided online remotely via a wired or wireless connection, for example, by an airline organisation via an online check-in process or through an application of an electronic device.

According to another aspect of the invention, there is provided a method of digitally authenticating a user's health status, performed at a verifying entity, the method comprising: receiving a certificate representative of health data associated with a user; verifying the certificate and, if the certificate is verified, providing a credential based on the verified certificate. The credential may comprise identity data associated with the user's identity.

The methods performed at any of the entities include the user device, the authenticator, and the verifying entity, may comprise one or more features of the methods of digitally authenticating a user's health status as described herein.

According to another aspect of the invention, there is provided computer program for carrying out the method according to embodiments described herein.

According to another aspect of the invention, there is provided a non-transitory computer-readable medium having stored thereon instructions that, when executed by a processor, cause the processor to perform the steps of the method according to embodiments described herein.

Arrangements described herein provide a decentralized identity system providing one or more of the following: provision of verifiable health data to the government, State, verifying entity or issuing authority; facilitation of issuers to join a decentralized ecosystem and ensure interoperability; compliance on data privacy regulations and provision of a secure authentication method (providing GDPR compliance including avoiding central databases, relying on strong cryptographic standards, and being open source); and be trusted by users (ensuring data cannot be controlled by a single organization).

From the foregoing, it will be appreciated that one or more of the entities including the user device, verifying entity, and authenticating entity may comprise a computer processor running one or more server processes for communicating with client devices. The server processes comprise computer readable program instructions for carrying out the operations of the present invention. The computer readable program instructions may be or source code or object code written in or in any combination of suitable programming languages including procedural programming languages such as C, object orientated programming languages such as C#, C++, Java, scripting languages, assembly languages, machine code instructions, instruction-set-architecture (ISA) instructions, and state-setting data.

The wired or wireless communication networks described above may be public, private, wired or wireless network. The communications network may include one or more of a local area network (LAN), a wide area network (WAN), the Internet, a mobile telephony communication system, or a satellite communication system. The communications network may comprise any suitable infrastructure, including copper cables, optical cables or fibres, routers, firewalls, switches, gateway computers and edge servers.

The one or more entities of the decentralised system described above may comprise a Graphical User Interface. Embodiments of the invention may include an on-screen graphical user interface. The user interface may be provided, for example, in the form of a widget embedded in a web site, as an application for a device, or on a dedicated landing web page. Computer readable program instructions for implementing the graphical user interface may be downloaded to the client device from a computer readable storage medium via a network, for example, the Internet, a local area network (LAN), a wide area network (WAN) and/or a wireless network. The instructions may be stored in a computer readable storage medium within the client device.

As will be appreciated by one of skill in the art, the invention described herein may be embodied in whole or in part as a method, a system, or a computer program product including computer readable instructions. Accordingly, the invention may take the form of an entirely hardware embodiment or an embodiment combining software, hardware and any other suitable approach or apparatus.

The computer readable program instructions may be stored on a non-transitory, tangible computer readable medium. The computer readable storage medium may include one or more of an electronic storage device, a magnetic storage device, an optical storage device, an electromagnetic storage device, a semiconductor storage device, a portable computer disk, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk.

Exemplary embodiments of the invention may be implemented as a circuit board which may include a CPU, a bus, RAM, flash memory, one or more ports for operation of connected I/O apparatus such as printers, display, keypads, sensors and cameras, ROM, a communications sub-system such as a modem, and communications media.

In addition, the above detailed description of embodiments of the invention are not intended to be exhaustive or to limit the invention to the precise form disclosed. For example, while processes or blocks are presented in a given order, alternative embodiments may perform routines having steps, or employ systems having blocks, in a different order, and some processes or blocks may be deleted, moved, added, subdivided, combined, and/or modified. Each of these processes or blocks may be implemented in a variety of different ways. Also, while processes or blocks are at times shown as being performed in series, these processes or blocks may instead be performed in parallel, or may be performed at different times.

The teachings of the invention provided herein can be applied to other systems, not necessarily the system described above. The elements and acts of the various embodiments described above can be combined to provide further embodiments.

While some embodiments of the inventions have been described, these embodiments have been presented by way of example only, and are not intended to limit the scope of the disclosure. Indeed, the novel methods and systems described herein may be embodied in a variety of other forms; furthermore, various omissions, substitutions and changes in the form of the methods and systems described herein may be made without departing from the spirit of the disclosure.

### BRIEF DESCRIPTION OF THE DRAWINGS

The invention will now be described in more detail, by way of example, with reference to the accompanying drawings, in which:
Figure 1 is a schematic diagram illustrating a user and user device embodying an aspect of the present invention;
Figure 2a is a schematic diagram illustrating a health data source, a user device, and a user embodying an aspect of the present invention;
Figure 2b is a schematic diagram illustrating a health data source, a user device, and a user embodying an aspect of the present invention;
Figure 2c is a schematic diagram illustrating a health data source, a user device, and a user embodying an aspect of the present invention;
Figure 3a is a schematic diagram illustrating a verifying entity, a user device, and a user embodying an aspect of the present invention;
Figure 3b is a schematic diagram illustrating a verifying entity, a user device, and a user embodying an aspect of the present invention;
Figure 3c is a schematic diagram illustrating a verifying entity, a user device, and a user embodying an aspect of the present invention;
Figure 4a is a schematic diagram illustrating an authenticator, a user device, and a user embodying an aspect of the present invention;
Figure 4b is a schematic diagram illustrating an authenticator, a user device, and a user embodying an aspect of the present invention;
Figure 5 is a schematic diagram illustrating a health data source, a verifying entity, an authenticator, and a user embodying an aspect of the present invention.

Like features are denoted by like reference numerals.

### DETAILED DESCRIPTION

In the following described example, a traveller wishing to use an airline service obtains a health certificate from an authorised health provider. The health certificate is stored in a digital wallet of an electronic device as a verifiable health credential that can be used as cryptographically signed proof of the existence of the issued health certificate. As the traveller proceeds on their journey, the status of the health certificate is updated and the verifiable health credential in their wallet is similarly updated with the new status in real-time. The traveller shares the updated verifiable health credential with the appropriate overseeing authority and a trusted traveller verifiable credential is offered if the verifiable health credential satisfies the governance rules. Upon acceptance of the trusted traveller verifiable credential, by the traveller, the traveller's digital wallet is updated with the trusted traveller verifiable credential, which can now be used as proof that the traveller has satisfied the overseeing authority's requirements. Even though the traveller has both the verifiable health credential and the trusted traveller credential in their digital wallet, only the trusted traveller verifiable credential needs to be presented for authentication as proof of compliance.

The system uses the Linux Foundation for Public Health, Cardea project ( https://www.lfph.io/projects/cardea/) as the open-source library, SITA is a founding member, for the technical framework. The Linux Foundation's open-source Indy Hyperledger project (https://www.hyperledger.org/use/hyperledger-indy) provides the capabilities of a distributed ledger. The system does not rely on databases to store any Personally Identifiable Information (Pll) data and as the verifiable credential is held by the traveller in their digital wallet (typically installed on an electronic device such as a smart phone) it does not contravene any GDPR requirements. The distributed ledger stores no actual data portions of the verifiable credential, instead, through the use of DIDs (Digital IDentifiers) and cryptographic keys the data is securely exchanged between consenting parties..

The overseeing authority issues the trusted traveller verifiable credential based upon a set of machine readable governance rules. The governance rules are the basis upon which a trusted traveller verifiable credential is issued, and in the system being described, a verifiable health credential which has a particular status is the defining trigger for the issuance of a trusted traveller verifiable credential. The system is flexible enough to add, modify and remove the governance rules in accordance with new or changing circumstances. In this respect, the governance framework will allow overseeing authorities to react to fluid situations. Verifiable credential revocation in real-time is supported. This may be in addition or alternate to other means of determining the validity of the verifiable credential, such as expiration date validation or positive/negative status checks.

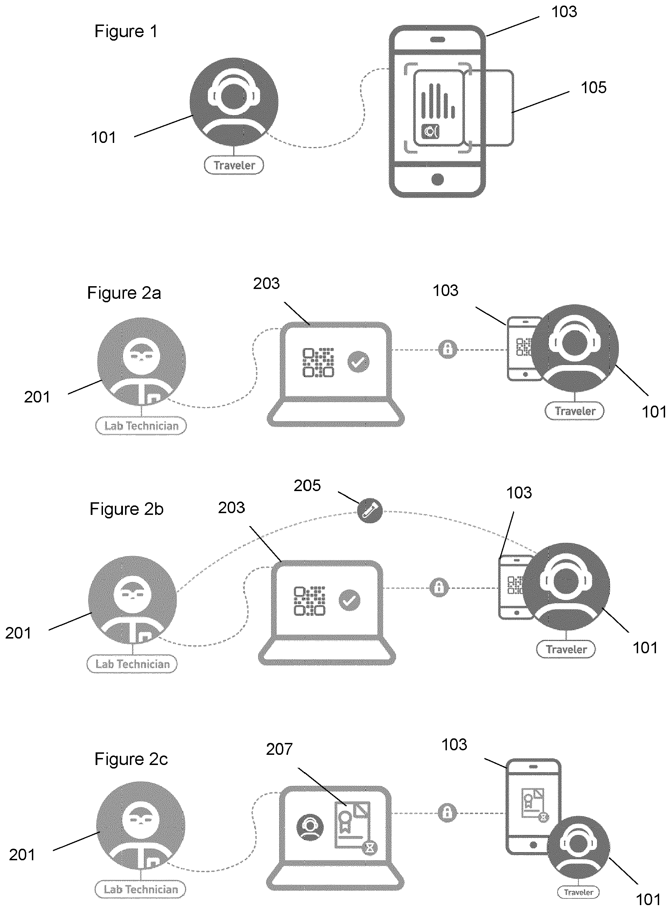

An example method for digitally authenticating a user's health status will now be described in more detail with reference to Figures 1 to 5.

Figure 1 illustrates a user 101, who in this example is a traveller intending to travel through an airport using an airline service. Initially, the user 101 downloads the application in accordance with described embodiments onto their electronic device, which in this example is a mobile phone. Having downloaded the application, the user 101 scans their passport 105 using a camera application on the mobile device 103. The application recognises identity data from the passport 105 and conveniently stores the identity data in the application. This may be basic identity information such as name and date of birth, or may include all identity information included on the passport. The user 101 may additionally or alternatively input identity information into the application manually through a user interface of the mobile device 105.

Figure 2a illustrates a health data source (which in this example is a testing lab 201) establishing an initial connection with the traveller 101. In particular, the mobile phone 103 of the user 101 establishes a secure connection with a lab device 203 of the testing lab 201. In this example, the secure connection is established by the mobile phone 103 generating a QR as a unique identifier, and the lab device 203 scanning and recognising the QR code. Establishing the secure connection also allows the testing lab 201 to verify the traveller's identity such that any subsequent testing can be correctly and securely linked to the traveller's identity. It will be appreciated that the lab device 203 could similarly produce the QR code and the mobile device 103 scan the QR code.

Having established the secure connection, the traveller 101 provides a sample 205 to the testing lab 201 as illustrated in Figure 2b. This may be, for example, a PCR test 205. Figure 2c then illustrates the testing lab 201 issuing a health test record or certificate 207 representative of the health data associated with the user through the established secure connection, the health data including information such as the test result or vaccination status. The certificate 207 is tied to the traveller's identity and, in this example, provides proof of having been tested. The traveller then awaits remote delivery of final results, which are initially pending. Conveniently, the mobile application (and the certificate 207) can be updated automatically once a result such as the PCR result is confirmed. For example, an in-app notification may appear for the traveller allowing them to accept the results, which are then stored in the on the mobile device 103 through the application.

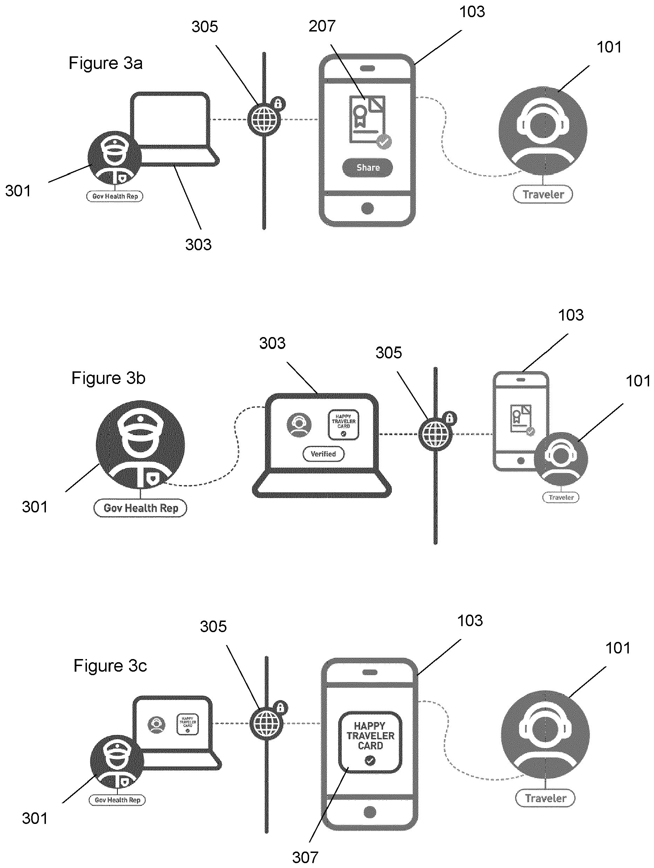

Figure 3a illustrates verification of the traveller's certificate 207 provided by the health data source. A secure online connection 305 is established between the traveller's device 103 and the verifier, which in this example is a government health department 301. In particular, it is the government health department 301 associated with the traveller's destination of travel. The certificate 207 issued by the testing lab 201 is provided to the government health department 301 via the secure online connection 305. Significantly, the certificate 207 is only representative of the health data associated with the traveller 103. That is, the certificate 207 provides only the relevant information needed by the government health department 301 to be able to verify the traveller's health data, and does not necessarily include the traveller's health data.

Having received the certificate 207, the government health department 301 is able to verify the certificate 207 and, if verified, provide a credential 307 based on the verified certificate 207 over the secure online connection 305. In this example, the verification process is automated based on a set of machine readable governance rules forming the basis of the verification. These rules may be requirements for permitting entry into the traveller's country of destination. For example, the rules may, for example, include determining whether or not one or more of: insurance has been purchased; consent has been given; a health question(s) has been completed; or health data has been verified. If the certificate 207 representative of the traveller's health data satisfies the appropriate rules, the government health department 301 verifies the certificate 207 and issues the credential 307 via the secure online connection 305 as illustrated in Figure 3c. In this example, the credential is termed a happy traveller card 307, signifying that the traveller is permitted to travel to their desired destination as verified by the government health department 301 of that country based on their requirements for travel. The credential 307 is stored in the mobile device 103 in a digital wallet of the mobile device 103. The credential 307 does not comprise the user's health data, but comprises identity data of the user allowing the credential to be associated with the user. As previously explained, in some embodiments, the verifying entity may comprise or work in conjunction with an issuing agent in order to issue the credential 307.

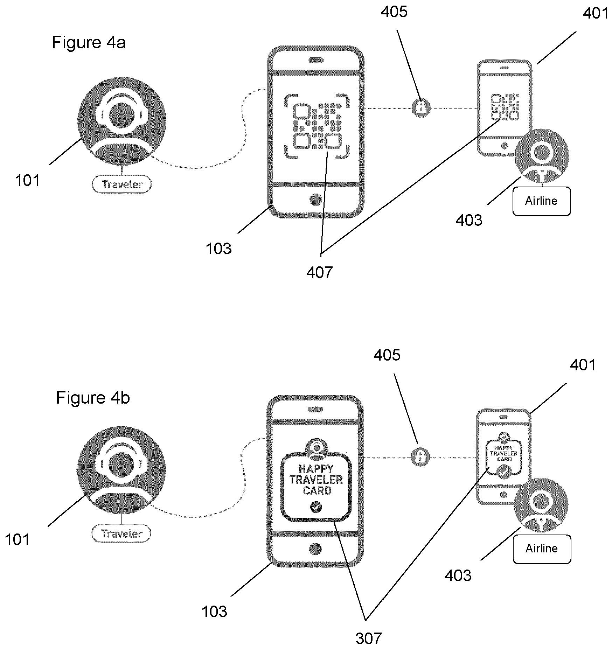

Figures 4a and 4b illustrate the authentication process of a user or traveller attempting to gain entry to, access, or use a service of a particular entity. In this example, the traveller 101 wishes to progress through an airport to use an airline service, but the method may equally be applied to a user wishing to gain entry to an entertainment venue or restaurant.

Figure 4a illustrates the traveller 101 establishing a secure peer-to-peer connection 405 with the authenticator, which here is an airline 403. In particular, a secure connection 405 is established between the mobile device 103 and an authenticator device 401 associated with the airline 403. To establish the secure connection, the authenticator device 401 generates a unique identifier 407 which is recognised by the mobile device 103. In this example, this process is performed remotely over an online internet connection accessed by both the mobile device 103 and authenticator device 401, the connection between the two parties being established over TCP/IP and the data being exchanged directly. All communications are encrypted using only the DID and verification keys known to each party.

Having established the secure connection 405, the credential 307 is provided to the airline 403 for authentication. At the time of authentication, where a traveller is requested to present proof of a credential, the data is exchanged over a securely established communication channel using the Aries Hyperledger protocols and DIDComm messaging standards. The traveller cannot alter the contents of the digital wallet as the data is cryptographically signed, so that any tampering of the data will render it invalid to the authenticator. The authenticator, once they have requested and received the proof presentation (a cryptographic representation of the verifiable credential), performs a verification lookup of the distributed ledger to verify the DID and credential definition components. The result of this distributed ledger lookup is used to determine the outcome of the authentication, that is, either authenticated or not. In this way, the traveller 101 is able to conveniently perform online check-in with their airline 403.

The open-source Aries Hyperledger protocols and DIDComm messaging standards are used to provide the lower layers of the trust framework and a set of governance rules are injected into the system to provide a level of trust ensuring that only authorised issuers have provided the credentials. Cryptographically secured messages over communication channels guarantee the privacy of all data exchanges between parties.

The separation of data (the verifiable credential information) and trust (who has issued the verifiable credential) is a feature of the system that allows an authenticator to be confident that a set of verifiable credentials has been provided to adequately satisfy the governance rules put in place by an appropriate overseeing authority (usually, but not limited to, a governmental agency).

The airline 403 confirms that the credential 307 is valid and has been issued by the government health department 301. The traveller 101 is then granted access to use the airline service. For example, as a result of authentication, the traveller is provided with a boarding pass permitting the traveller 101 to board an aircraft.

The methods described herein use the term authentication for the process of an entity determining whether a user or traveller may be permitted entry, access, or use of a service. However, it will be appreciated that this may be termed verification, verifying the user's health status from the credential issued by the government authority. The authenticator may similarly be termed a verifier.

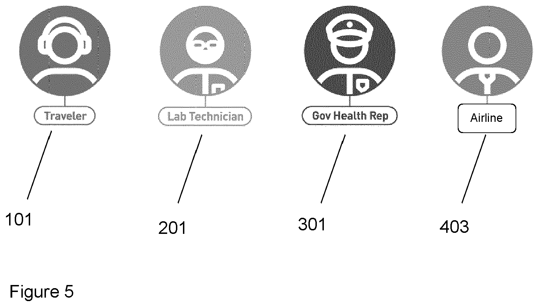

Figure 5 illustrates each of the four entities involved in the method of digitally authenticating a user's health status. Methods of digitally authenticating a user's health status according to techniques of this disclosure comprise sending a certificate 207 representative of health data associated with a user 101 to a verifying entity 301; the verifying entity 301 carrying out verification on the certificate 207 and, if the certificate 207 is verified, providing a credential 307 based on the verified certificate 207; and authenticating the user 101 associated with the health data based on the credential 307.

Performed at a user device 101, the method of digitally authenticating the user's health status comprises the user device 101: sending a certificate 207 representative of health data associated with a user 101; receiving a credential 307 based on the certificate 207 being verified; and providing the credential 307 for authentication.

Performed at an authenticator 403, the method of digitally authenticating a user's health status comprises the authenticator 403: receiving a credential 307 representative of a verified certificate 207 representing health data associated with a user 101; and authenticating the user 101 associated with the health data based on the credential 307.

Performed at the verifying entity 301, the method of digitally authenticating a user's health status comprises: receiving a certificate 207 representative of health data associated with a user 101; verifying the certificate 207 and, if the certificate 207 is verified, providing a credential 307 based on the verified certificate 207.

Methods described herein employ a trust framework as described in the Trust Over IP Foundation's (https://trustoverip.org/) principles of digital trust. The framework embodies the use of verifiable credentials that may be held by an individual in a cryptographically secure digital wallet. The verifiable credentials can be self-asserted or issued by a trusted issuing agent, and authenticated by interested parties. The digital trust framework uses a governance framework to ensure the parties involved in the issuance of the credentials to be subsequently authenticated can be trusted. Each role (verifying entity and issuing agent, user, and authenticator) operates independently. Governance provides the basis for trusted decisions based on legislation, policy, regulations, process, and reputation.

Privacy is preserved, and security is enhanced via open standards which enable both independence and scale. This may be provided by one or more of: private cryptographic peer to peer connections (surveillance resistant); standard cryptographic data exchange protocols between peers (impersonation resistant); the holder is in control of providing the verifier with proof of data (privacy preserving); the verifier verifies data from the holder without contacting the issuer (surveillance resistant); and the verifier determines what data they need to verify from what set of issuers (fraud resistant).

The described example embodiment comprises online authentication. A particularly advantageous feature of techniques of this disclosure is that it is possible to perform offline verification, where both the holder and verifier are not connected to the internet or network. The availability of offline verification is predicated on the fact that in order to receive a verifiable credential the traveller 101 must have, at one stage, been online. It is when the traveller 101 is online that certain, crucial, records can be obtained and stored in a cache on traveller's device 103. Similarly, the authenticator 403 must have, at one previous point in time, been online and retrieved and cached the relevant records. The records that may be cached locally are the schema definition and the credential definition, both of which are held on the distributed ledger. The traveller and authenticator can perform an authentication over a communication channel using the cached versions of the records instead of having to call out to the distributed ledger. Establishment of the communication channel is achieved through the use of a peer-to-peer connection such as Bluetooth connectivity, rather than TCP/IP as no network is available.

The use of open-source standards, such as W3C verifiable credentials, Aries/Usra Hyperledger, DIDComm, and Trust over IP ensures a level of interoperability with other solutions built upon the same foundations. As an example, a verifiable credential issued by the system can be accepted, stored and presented using a digital wallet provided by an enterprise other than the one created by system described. If the Aries protocols and DIDComm messaging standards are adhered to, there is no lock-in to using a specific digital wallet implementation.

Embodiments of the present invention have been described. It will be appreciated that variations and modifications may be made to the described embodiments within the scope of the present invention.

Although the above example has been described with reference to a system for use in the aviation industry, it will be clear from the foregoing explanation embodiments of the invention may advantageously be used in any environment where a health status check needs to be performed, such as a restaurant, cinema, shopping centre, or transportation hub such as rail or bus station.

## Claims

1. A method of digitally authenticating a user's health status, the method comprising:
sending a certificate representative of health data associated with a user to a verifying entity;
the verifying entity carrying out verification on the certificate and, if the certificate is verified, providing a credential based on the verified certificate; and
authenticating the user associated with the health data based on the credential.

2. The method of claim 1 wherein the credential comprises identity data associated with the user's identity.

3. The method of claim 2 wherein the credential does not comprise the user's health data.

4. The method of any preceding claim wherein the verification is based on one or more rules, and preferably the method further comprises updating at least one of the one or more rules in response to a change in one or more criteria, and more preferably wherein the one or more rules comprise determining whether or not one or more of: the user has purchased insurance; the user has given consent; the user has completed one or more health questions; and health data has been verified.

5. The method of claim 2 wherein the identity data comprises one or more of: biographic data associated with the user; biometric data associated with the user; and reference to a travel document.

6. The method of any preceding claim further comprising applying a cryptographic signature to the health data to prevent alteration of the health data.

7. The method of any preceding claim further comprising receiving the certificate representative of health data from a health data source, and preferably wherein the receiving the certificate comprises establishing a secure link between the health data source and the user by a unique identifier associated with the user.

8. The method of any preceding claim further comprising, if the certificate is not verified, determining the user as non-compliant and not authenticating the user.

9. The method of any preceding claim further comprising revoking the credential if it is determined that the certificate should no longer be verified.

10. The method of any preceding claim further comprising deriving one or more additional credentials from a digital travel card embedded on the credential.

11. The method of any preceding claim wherein the verifying entity is a government authority; and/or the authenticating is performed by an authenticator, and preferably the authenticator is an airline organisation.

12. A method of digitally authenticating a user's health status, performed at a user device, the method comprising the user device:
sending a certificate representative of health data associated with a user;
receiving a credential based on the certificate being verified; and
providing the credential for authentication.

13. A method of digitally authenticating a user's health status, performed at an authenticator, the method comprising the authenticator:
receiving a credential representative of a verified certificate representing health data associated with a user; and
authenticating the user associated with the health data based on the credential.

14. A method of digitally authenticating a user's health status, performed at a verifying entity, the method comprising:
receiving a certificate representative of health data associated with a user;
verifying the certificate and, if the certificate is verified, providing a credential based on the verified certificate.

15. A computer program for carrying out the method of any of claims 1 to 14.
